(19) Europäisches Patentamt

European Patent Office

Office européen des brevets

(11) Publication number: **0 323 270 B1**

(12) # EUROPEAN PATENT SPECIFICATION

(45) Date of publication of patent specification: **13.04.94**

(51) Int. Cl.5: **C07C 29/04**, C07C 41/06, C07C 41/09

(21) Application number: **88312427.3**

(22) Date of filing: **30.12.88**

(54) Process for the catalytic hydration of olefins.

(30) Priority: **30.12.87 US 139567**

(43) Date of publication of application:
**05.07.89 Bulletin 89/27**

(45) Publication of the grant of the patent:
**13.04.94 Bulletin 94/15**

(84) Designated Contracting States:
**BE DE FR GB IT NL SE**

(56) References cited:
**EP-A- 0 130 697          EP-A- 0 210 793**
**EP-A- 0 213 884          EP-A- 0 323 137**
**EP-A- 0 323 268          US-A- 4 131 750**
**US-A- 4 214 107**

(73) Proprietor: **MOBIL OIL CORPORATION**
**3225 Gallows Road**
**Fairfax, Virginia 22037-0001(US)**

(72) Inventor: **Bell, Weldon K.**
**428 Burd Street**
**Pennington, NJ 08534(US)**
Inventor: **Huang, Tracy J.**
**9 Woodfield Lane**
**Lawrenceville, NJ 08648(US)**
Inventor: **Haag, Werner O.**
**38 Pine Knoll Drive**
**Lawrenceville, NJ 08648(US)**
Inventor: **Sorensen, Charles M.**
**3303 Hermitage Road**
**Wilmington, DE 19810(US)**

(74) Representative: **Jones, Alan John et al**
**CARPMAELS & RANSFORD**
**43 Bloomsbury Square**
**London, WC1A 2RA (GB)**

EP 0 323 270 B1

**Description**

This invention relates to a process for the catalytic hydration of olefins to provide alcohols, ethers and their mixtures. More particularly, the invention relates to a process for the hydration of light olefins such as ethylene, propylene, butenes, pentenes, hexenes, heptenes, etc., and their mixtures to provide a mixture of alcohol(s) and ether(s) employing the acidic form of a synthetic large pore crystalline material, or zeolite, as catalyst. The co-produced alcohols and ethers are useful, inter alia, as high octane blending stocks for gasolines.

There is a need for an efficient catalytic process to manufacture alcohols and ethers from light olefins thereby augmenting the supply of high octane blending stocks for gasoline. Lower molecular weight alcohols and ethers such as isopropyl alcohol (IPA) and diisopropyl ether (DIPE) are in the gasoline boiling range and are known to have a high blending octane number. In addition, by-product propylene from which IPA and DIPE can be made is usually available in a fuels refinery. The petrochemicals industry also produces mixtures of light olefin streams in the $C_2$ to $C_7$ molecular weight range and the conversion of such streams or fractions thereof to alcohols and/or ethers can also provide products useful as solvents and as blending stocks for gasoline.

The catalytic hydration of olefins to provide alcohols and ethers is a well-established art and is of significant commercial importance. US-A-4,214,107 discloses hydration of lower olefins, in particular, propylene, over a crystalline aluminosilicate zeolite catalyst having a silica to alumina ratio of at least 12 and a Constraint Index of from 1 to 12, e.g. HZSM-5 type zeolite, to provide the corresponding alcohol, essentially free of ether and hydrocarbon by-product.

According to US-A-4,499,313 (≡ EP-A-130 697) an olefin is hydrated to the corresponding alcohol in the presence of H-mordenite or H-Y each having a silica-alumina molar ratio of 20 to 500. The use of such a catalyst is said to result in higher yields of alcohol than olefin hydration processes which employ conventional solid acid catalysts. Use of the catalyst is said to offer the advantage over ion-exchange type olefin hydration catalysts of not being restricted by the hydration temperature. Reaction conditions employed in the process include a temperature of from 50-300°C, preferably 100-250°C, a pressure of 5 to 200 kg/cm$^2$ to maintain liquid phase or gas-liquid multi-phase conditions and a mole ratio of water to olefin of from 1 to 20. The reaction time can be 20 minutes to 20 hours when operating batchwise and the liquid hourly space velocity (LHSV) is usually 0.1 to 10 in the case of continuous operation.

EP-A-210,793 describes an olefin hydration process employing a medium pore zeolite as hydration catalyst. Specific catalysts mentioned are Theta-1, said to be preferred, ferrierite, ZSM-22, ZSM-23 and NU-10.

According to the present invention a process for converting light olefin(s) to a mixture of alcohol(s) and ether(s) comprises contacting a feed containing at least one light olefin with water in the vapour and/or liquid phase at a temperature of from 100 to 230°C, a total pressure of at least 5 and up to 300 atmospheres (bar) and a mole ratio of water to total olefin of less than 1 in the presence of a catalyst comprising zeolite beta, the resulting mixture of alcohol(s) and ether(s) containing no more than 25 weight percent oligomers.

The mixture of alcohol(s) and ether(s) resulting from the foregoing process are advantageously employed as a blending component for gasoline, as a cosolvent for methanol to be incorporated into gasoline, and many other applications.

The present invention is applicable to the hydration of individual light olefins and mixtures of olefins contained in refinery streams such as gas plant off-gas containing ethylene and propylene, naphtha cracker off-gas containing light olefins, fluidized catalytic cracked (FCC) light gasoline containing pentenes, hexenes and heptenes, refinery FCC propane/propylene streams, etc. For example, a typical FCC light olefin stream possesses the following composition:

| Typical Refinery FCC Light Olefin Composition | | |
|---|---|---|
| | Wt.% | Mole% |
| Ethane | 3.3 | 5.1 |
| Ethylene | 0.7 | 1.2 |
| Propane | 14.5 | 15.3 |
| Propylene | 42.5 | 46.8 |
| Isobutane | 12.9 | 10.3 |
| n-Butane | 3.3 | 2.6 |
| Butenes | 22.1 | 18.3 |
| Pentanes | 0.7 | 0.4 |

The process of the invention is especially applicable to the conversion of propylene and propylene-containing streams to mixtures of IPA and DIPE.

For acceptable results to be achieved, the conditions of olefin hydration must be maintained within relatively narrow limits. Suitable operating conditions are a temperature of 100 to 230°C, preferably from 120 to 220°C and most preferably from 140 to 220°C, a total system pressure of at least 5 atm (bar), preferably at least 20 atm (bar) and more preferably at least 40 atm (bar), a water to total olefin mole ratio of 0.1 to less than 1.0, preferably from 0.2 to 0.8 and most preferably from 0.3 to 0.7.

Those skilled in the art will recognize that selection of specific conditions for a particular feed will influence product distribution. It will also be appreciated that the precise conditions selected will in some measure reflect the nature of the olefin feed, isoolefins generally requiring milder process conditions than straight chain olefins.

The olefin hydration process of this invention can be carried out under liquid phase, vapour phase or mixed vapour-liquid phase conditions in batch or continuous manner using a stirred tank reactor or fixed bed flow reactor, e.g., trickle-bed, liquid-up-flow, liquid-down-flow, counter-current, co-current, etc. Reaction times of from about 20 minutes to about 20 hours when operating in batch and an LHSV of from about 0.1 to about 10 when operating continuously are suitable. It is generally preferable to recover any unreacted olefin and recycle it to the reactor.

When seeking to maximise the production of ether by the hydration of olefin, the aqueous product effluent from the olefin hydration reactor containing both alcohol and ether olefin hydration products can be introduced into a separator, e.g., a distillation column, for recovery of ether. The dilute aqueous solution of alcohol may be then introduced into a second separator, e.g., another distillation column, where a water/alcohol azeotrope is recovered. A fraction of the azeotrope may be fed into a dehydration reactor of conventional or otherwise known type and operation to provide a further quantity of ether which can be combined with the ether previously recovered from the olefin hydration reactor. By blending various product streams, almost any ratio of alcohol/ether can be obtained. When alcohol/ether mixtures are to be used as gasoline blending stocks, this capability for adjusting the ratios of alcohol to ether offers great flexibility in meeting the octane requirements for given gasoline compositions. Regulatory considerations aside, alcohol/ether mixtures, e.g., IPA/DIPE mixtures, can constitute up to about 20 weight percent or so of the gasoline to which they are added.

A particularly advantageous procedure for producing mixtures of alcohol and ether, and in particular IPA and DIPE, from the hydration of an olefin-containing feed (a propylene-containing feed in the case of IPA/DIPE mixtures) comprises co-feeding a fresh propane/propylene-containing feed (readily available in many petroleum refineries) and fresh water, together with recycled unreacted propylene and recycled water from a decanter, into a hydration reactor. The reactor effluent is passed to a separator unit with propane and unconverted propylene being recycled to the reactor, part of the gaseous mixture being purged in order to avoid build-up of propane in the recycle loop. The liquid products from the separator unit are introduced to a distillation unit where an azeotropic mixture of IPA, DIPE, water and propylene oligomers (mostly $C_6$ olefin) is distilled off and, following cooling, is introduced into a decanter in which phase separation takes place. The upper layer contains mostly DIPE, e.g., 90 weight percent or more, and relatively little water, e.g., 1 weight percent or so. The lower layer is largely water containing negligible quantities of IPA and DIPE. The quantity of the decanter overhead which is recycled can be regulated so as to control the water content in the final product. The bottom fraction from the distillation unit, mainly IPA, is combined with DIPE in the decanter overhead to provide the final IPA/DIPE mixture.

Where it is desired to separate out the alcohol from an alcohol/ether mixture and thus provide essentially pure ether, the propylene component of a mixed propane/propylene feed advantageously

3

undergoes hydration in the presence of the hydration catalyst in a hydration reactor with the effluent therefrom being passed to a separator operating below the olefin hydration reaction temperature. There, two liquid phases form, the aqueous phase being removed and recycled to the hydration reactor. The hydrocarbon-rich phase is flashed to a lower pressure to effect separation of the unreacted $C_3$ components. The flashed product, now containing a substantial amount of IPA product, is introduced to a distillation unit operated at or below atmospheric pressure to effect further purification of the DIPE. The azeotropic IPA, DIPE and water overhead product containing a small amount of propylene oligomer is condensed and thereafter contacted with reactor feed water. The resulting phase separation provides a DIPE product containing at most negligible amounts of IPA and water, e.g., 1.0 weight percent and 0.5 weight percent of these materials, respectively. The remaining aqueous phase can be recycled to the reactor.

For purposes of this specification the term "zeolite" includes the class of porotectosilicates, i.e., porous crystalline silicates which contain silicon and oxygen atoms as the major framework elements. Other elements can be present in minor amounts, usually less than 14 mole %, and preferably less than 4 mole %, these including aluminium, gallium, iron, boron, and the like, with aluminium being preferred.

The framework silica-to-alumina mole ratio is meant to represent, as closely as possible, the ratio of silica to alumina in the rigid anionic framework of the zeolite crystal and to exclude any alumina which may be present in any binder material optionally associated with the zeolite or present in cationic or other form within the channels of the zeolite. Although forms of the zeolite with a silica-to-alumina mole ratio of greater than about 7 are useful, it is preferred to use forms of much higher silica-to-alumina mole ratios, i.e., ratios of at least about 20:1 and preferably greater than about 40:1, e.g., 100:1 and even higher. In addition zeolite beta substantially free of aluminium, i.e., having a silica-to-alumina mole ratio up to and including infinity, is useful and can even be preferable in some cases.

Zeolite beta for use herein will generally possess an alpha value of at least about 1, preferably at least about 10 and more preferably at least about 100. (The alpha test is described in J. Catalysis, 6, pp. 278-287 (1966) and J. Catalysis, 61, pp. 390-396 (1980).) Zeolites of low acidity (alpha values of less than about 200) can be prepared by a variety of techniques including (a) synthesising a zeolite with a high silica/alumina ratio, (b) steaming, (c) steaming followed by dealuminsation and (d) substituting aluminium with other species. For example, in the case of steaming, the zeolite can be exposed to steam at elevated temperatures ranging from about 500 to about 1200°F (260 to 649°C) and preferably from about 750 to about 1000°F (339 to 538°C). This treatment can be accomplished in an atmosphere of 100% steam or an atmosphere consisting of steam and a gas which is substantially inert to the zeolite. A similar treatment can be accomplished at lower temperatures employing elevated pressure, e.g., at from about 350 to about 700°F (177 to 371°C) with from about 10 to 200 atmospheres (10.14 to 202.76 bar). Aside from or in addition to any of the foregoing procedures, the surface acidity of the zeolite can be eliminated or reduced by treatment with bulky reagents as described in US-A-4,520,221.

In practising the olefin hydration process of the present invention, it can be advantageous to incorporate the zeolite beta into some other material, i.e., a matrix or binder, which is resistant to the temperature and other conditions employed in the process. Useful matrix materials include both synthetic and naturally-occurring substances, e.g,, inorganic materials such as clay, silica and/or metal oxides. Such materials can be either naturally-occurring or can be obtained as gelatinous precipitates or gels including mixtures of silica and metal oxides. Naturally-occurring clays which can be composited with the zeolite include those of the montmorillonite and kaolin families, which families include the sub-bentonites and the kaolins commonly known as Dixie, McNamee-Georgia and Florida clays or others in which the main mineral constituent is haloysite, kaolinite, dickite, nacrite or anauxite. Such clays can be used in the raw state as originally mined or initially subjected to calcination, acid treatment or chemical modification.

In addition to the foregoing materials, the zeolite can be composited with a porous matrix material such as carbon, alumina, silica, titania, zirconia, silica-alumina, silica-magnesia, silica-zirconia, silica-thoria, silica-beryllia and silica-titania, as well as ternary oxide compositions such as silica-alumina-thoria, silica-alumina-zirconia, silica-alumina-magnesia and silica-magnesia-zirconia. The matrix can be in the form of a cogel. The relative proportions of zeolite component and matrix material, on an anhydrous basis, can vary widely with the zeolite content ranging from between about 1 to about 99 wt%, and more usually in the range of about 5 to about 90 wt%, of the dry composite.

In some cases, it may be advantageous to provide the zeolite beta as an extrudate bound with a low acidity refractory oxide binder. Such an extrudate may be prepared by forming a homogeneous mixture of the zeolite, water and a low acidity refractory oxide binder, e.g., silica, which contains at least an extrusion-facilitating amount of the binder in a colloidal state and which is substantially free of added alkali metal base and/or base salt, into an extrudable mass. The mass is extruded and the resulting extrudate dried and calcined.

4

The original cations associated with the zeolite can be replaced by a wide variety of other cations according to techniques well known in the art, e.g., by ion-exchange. Typical replacing cations include hydrogen, ammonium, alkyl ammonium and metal cations, and their mixtures. Metal cations can also be introduced into the zeolite. In the case of replacing metallic cations, particular preference is given to metals of Groups IB to VIII of the Periodic Table, including, by way of example, iron, nickel, cobalt, copper, zinc, palladium, calcium, chromium, tungsten, molybdenum, rare earth metals. These metals can also be present in the form of their oxides.

The following examples are illustrative of the olefin hydration process of the present invention.

EXAMPLE 1

Zeolite Beta (hydrogen form; binder free) was employed in a number of hydration runs under varying conditions.

The conditions of the hydration runs and the results thereof are set forth in Table 1 as follows:

Table 1:  Propylene Hydration Under Varying Conditions

| Hydration Conditions | RUN | | | | | |
| | 1 | 2 | 3 | 4 | 5 | 6 |
| Temp., $^{o}$C | 201 | 202 | 202 | 200 | 202 | 202 |
| Pressure, psi (bar) | 255 (17.6) | 255 | 255 | 255 | 255 | 255 |
| Water:Propylene Mole Ratio | 0.99 | 0.93 | 0.99 | 4.74 | 1.00 | 1.17 |
| LHSV, total cat. | 18.21 | 9.28 | 5.04 | 4.26 | 2.51 | 0.35 |
| WHSV, total cat. | 20.144 | 10.197 | 5.574 | 5.956 | 2.783 | 0.393 |
| **CONVERSION, %** | | | | | | |
| Propylene | 3.98 | 6.58 | 7.79 | 10.37 | 9.13 | 9.06 |
| Water | 4.27 | 7.46 | 7.84 | 2.58 | 8.78 | 11.08 |
| IPA | 3.7 | 6.5 | 6.7 | 10.1 | 7.5 | 8.3 |
| DIPE | 0.2 | 0.0 | 0.0 | 0.1 | 0.0 | 0.2 |
| Hydrocarbon (HC) (propylene oligomer) | 0.1 | 0.1 | 1.0 | 0.1 | 1.7 | 0.6 |
| IPA g/hr/l cat. | 445.7 | 380.0 | 219.0 | 179.5 | 122.0 | 20.5 |
| HC g/hr/l cat. | 9.4 | 4.4 | 23.6 | 1.5 | 18.9 | 1.0 |
| DIPE g/hr/l cat. | 16.8 | 0.5 | 0.1 | 2.0 | 0.1 | 0.4 |
| IPA/IPA+HC+DIPE | 0.944 | 0.987 | 0.902 | 0.981 | 0.865 | 0.935 |

As these data show, by operating at a high space velocity at near equilibrium conversion, hydrocarbon coproduction is minimised at about 5%.

EXAMPLE 2

This example illustrates the effect of the feed water:propylene mole ratio on the coproduction of hydrocarbon employing zeolite Beta (bound with 35 weight percent alumina).

**Table 2: Effect of Water:Propylene Mole Ratio**

| Hydration Conditions | RUN 1 | 2 | 3 | 4 | 5 | 6 | 7 | 8 |
|---|---|---|---|---|---|---|---|---|
| Temp., °C | 162 | 161 | 161 | 162 | 162 | 162 | 205 | 214 |
| Pressure, psi (bar) | 1000 (70) | 1000 | 1000 | 1000 | 1000 | 1000 | 255 | 255 |
| Water:Propylene Mole Ratio | 0.48 | 2.28 | 3.57 | 0.74 | 0.57 | 1.76 | 1.00 | 0.98 |
| LHSV, total cat. | 0.48 | 0.66 | 0.35 | 0.13 | 0.49 | 0.61 | 14.35 | 19.77 |
| WHSV, total cat. | 0.495 | 0.823 | 0.469 | 0.141 | 0.514 | 0.728 | 13.601 | 21.822 |
| **CONVERSION BASED ON PRODUCTS** | | | | | | | | |
| Propylene | 30.91 | 31.41 | 60.30 | 62.63 | 25.52 | 24.52 | 23.39 | 4.79 |
| Water | 62.19 | 11.36 | 14.48 | 54.21 | 33.67 | 12.10 | 0.00 | 2.56 |
| IPA | 11.8 | 17.8 | 31.1 | 21.9 | 11.8 | 17.6 | 0.0 | 2.2 |
| DIPE | 17.6 | 12.4 | 24.1 | 36.7 | 12.8 | 6.9 | 0.0 | 0.0 |
| Hydrocarbon (HC) | 1.5 | 1.2 | 5.0 | 4.0 | 1.0 | 0.0 | 23.4 | 2.6 |
| IPA g/hr/l cat. | 38.3 | 56.3 | 44.4 | 17.2 | 36.6 | 55.7 | 0.0 | 277.9 |
| HC g/hr/l cat. | 3.4 | 2.7 | 5.0 | 2.2 | 2.1 | 0.0 | 1971.5 | 232.1 |
| DIPE g/hr/l cat. | 48.4 | 33.1 | 29.2 | 24.4 | 33.8 | 18.5 | 0.0 | 0.0 |
| IPA/IPA+HC+DIPE | 0.425 | 0.611 | 0.565 | 0.392 | 0.505 | 0.750 | 0.000 | 0.545 |

As these data show, water strongly inhibits polymerisation. For a pure propylene feed, hydrocarbon production was 14 times that for an equimolar water:propylene feed (Runs 7 and 8). It is unnecessary to employ a large excess of water as the latter does not exert a proportional suppression of hydrocarbon coproduction but has the disadvantage of requiring greater water recycle capability.

EXAMPLE 3

Data set forth below in Table 3 below show the oxygenate selectivities for a mixed propylene/butene feed hydrated in the presence of zeolite Beta extrudate bound with 17 weight percent colloidal silica. The feed consisted of the following components: water/propane/propylene/1-butene/n-butane in the respective weight ratios of 11:9:24:32:23. Reaction conditions were: 330°F (166°C), 1750 psi (121.7 bar) and 0.5 olefin WHSV.

Table 3

| Conversion of Propylene/Butene Mixtures to Alcohols and Ethers | |
|---|---|
| Alcohol/ether Product | % Selectivity |
| 2-propanol | 41 |
| 2-butanol | 29 |
| $C_8$ ethers | 2 |
| diisopropyl ether | 12 |
| isopropyl, 2-butyl ether | 10 |

**Claims**

1. A process for converting light olefins to alcohols and/or ethers which comprises contacting a feed containing at least one light olefin with water in the vapour and/or liquid phase at a temperature of 100 to 230°C, a pressure of 5 to 300 atmospheres (bar) and a mole ratio of water to total olefin of less than 1 in the presence of a catalyst comprising zeolite beta, the product alcohols and/or ethers containing no more than 25 weight percent oligomers.

2. A process according to claim 1 wherein the feed contains a mixture of light olefins.

3. A process according to claim 1 or claim 2 wherein the olefins contain from two to seven carbon atoms.

4. A process according to any preceding claim wherein the feed contains ethylene, propylene, a butene, a pentene, a hexane and/or a heptene.

**5.** A process according to any preceding claim wherein the feed is gas plant off-gas containing ethylene and propylene.

**6.** A process according to any preceding claim wherein the feed is naphtha cracker off-gas containing light olefins.

**7.** A process according to any of claims 1 to 4 wherein the feed is light catalytic cracked gasoline containing pentenes, hexenes and heptenes.

**8.** A process according to any preceding claim wherein the temperature is 120° to 220°C.

**9.** A process according to claim 8 wherein the temperature is 140° to 200°C.

**10.** A process according to any preceding claim wherein the pressure is 15 to 250 atm (bar).

**11.** A process according to claim 10 wherein the pressure is 20 to 200 atm (bar).

**12.** A process according to any preceding claim wherein the mole ratio of water to total olefin is 0.1 to 0.9.

**13.** A process according to claim 12 wherein the mole ratio of water to total olefin is from 0.2 to 0.8.

**14.** A process according to any preceding claim wherein the framework silica to alumina ratio of the zeolite beta is greater than 20.

**15.** A process according to any preceding claim wherein the framework silica to alumina ratio of the zeolite beta is greater than 200.

**16.** A process according to any preceding claim wherein the zeolite beta is composited with a binder.

**17.** A process according to claim 16 wherein the binder is silica and/or alumina.

**18.** A process according to any preceding claim wherein the zeolite beta is composited with at least an extrusion-facilitating amount of a low acidity refractory oxide binder in the colloidal state which is substantially free of alkali metal base or basic salt.

**19.** A process according to any preceding claim wherein the catalyst further comprises a co-catalyst effective for the hydration of light olefin.

**20.** A process according to any preceding claim wherein unreacted olefin is recycled.

**21.** A process according to any preceding claim wherein the product contains no more than 10 weight percent oligomers.

**22.** A process according to any preceding claim wherein the feed contains at least 20 weight percent propylene.

**23.** A process according to claim 22 wherein the feed is a refinery FCC light olefin stream.

**Patentansprüche**

**1.** Verfahren zur Umwandlung leichter Olefine in Alkohole und/oder Ether, das den Kontakt einer Beschickung, die mindestens ein leichtes Olefin enthält, mit Wasser in der Dampfphase und/oder der flüssigen Phase bei einer Temperatur von 100 bis 230°C, einem Druck von 5 bis 300 Atmosphären (bar) und einem Molverhältnis von Wasser zum gesamten Olefin von weniger als 1 in Gegenwart eines Katalysators umfaßt, der Zeolith Beta umfaßt, wobei das Produkt in Form von Alkoholen und/oder Ethern nicht mehr als 25 Gew.-% Oligomere enthält.

**2.** Verfahren nach Anspruch 1, worin die Beschickung eine Mischung von leichten Olefinen enthält.

**3.** Verfahren nach Anspruch 1 oder 2, worin die Olefine von 2 bis 7 Kohlenstoffatome enthalten.

**4.** Verfahren nach einem der vorstehenden Ansprüche, worin die Beschickung Ethylen, Propylen, Buten, Penten, Hexan und/oder Hepten enthält.

**5.** Verfahren nach einem der vorstehenden Ansprüche, worin die Beschickung ein Abgas einer Gasanlage ist, das Ethylen und Propylen enthält.

**6.** Verfahren nach einem der vorstehenden Ansprüche, worin die Beschickung Abgas einer Benzincrackanlage ist, das leichte Olefine enthält.

**7.** Verfahren nach einem der Ansprüche 1 bis 4, worin die Beschickung katalytisch gecracktes Leichtbenzin ist, das Pentene, Hexene und Heptene enthält.

**8.** Verfahren nach einem der vorstehenden Ansprüche, worin die Temperatur 120 bis 220 °C beträgt.

**9.** Verfahren nach Anspruch 8, worin die Temperatur 140 bis 200 °C beträgt.

**10.** Verfahren nach einem der vorstehenden Ansprüche, worin der Druck 15 bis 250 atm (bar) beträgt.

**11.** Verfahren nach Anspruch 10, worin der Druck 20 bis 200 atm (bar) beträgt.

**12.** Verfahren nach einem der vorstehenden Ansprüche, worin das Molverhältnis von Wasser zum gesamten Olefin 0,1 bis 0,9 beträgt.

**13.** Verfahren nach Anspruch 12, worin das Molverhältnis von Wasser zum gesamten Olefin von 0,2 bis 0,8 beträgt.

**14.** Verfahren nach einem der vorstehenden Ansprüche, worin das Siliciumdioxid/Aluminiumoxid-Gitterverhältnis des Zeolith Beta größer als 20 ist.

**15.** Verfahren nach einem der vorstehenden Ansprüche, worin das Siliciumdioxid/Aluminiumoxid-Gitterverhältnis des Zeolith Beta größer als 200 ist.

**16.** Verfahren nach einem der vorstehenden Ansprüche, worin der Zeolith Beta mit einem Bindemittel zusammengesetzt ist.

**17.** Verfahren nach Anspruch 16, worin das Bindemittel Siliciumdioxid und/oder Aluminiumoxid ist.

**18.** Verfahren nach einem der vorstehenden Ansprüche, worin der Zeolith Beta mit mindestens einer die Extrusion fördernden Menge eines hitzebeständigen Oxidbindemittels mit geringer Acidität in kolloidalem Zustand verbunden ist, das im wesentlichen ohne Alkalimetallbase oder basisches Salz ist.

**19.** Verfahren nach einem der vorstehenden Ansprüche, worin der Katalysator außerdem einen gleichzeitig vorhandenen Katalysator umfaßt, der für die Hydrierung des leichten Olefins effektiv ist.

**20.** Verfahren nach einem der vorstehenden Ansprüche, worin das unreagierte Olefin rezirkuliert wird.

**21.** Verfahren nach einem der vorstehenden Ansprüche, worin das Produkt nicht mehr als 10 Gew.-% Oligomere enthält.

**22.** Verfahren nach einem der vorstehenden Ansprüche, worin die Beschickung mindestens 20 Gew.-% Propylen enthält.

**23.** Verfahren nach Anspruch 22, worin die Beschickung ein leichter Olefinstrom der FCC-Raffinerie ist.

**Revendications**

1.  Un procédé de conversion d'oléfines légères en alcools et/ou en éthers qui consiste à mettre en contact une charge contenant au moins une oléfine légère avec de l'eau en phase vapeur et/ou liquide à une température comprise entre 100 et 230 °C, une pression comprise entre 5 et 300 atmosphères (bar) et un rapport molaire eau/oléfine totale inférieur à 1, en présence d'un catalyseur comprenant la zéolite bêta, le mélange alcools et/ou éthers produit ne contenant pas plus de 25% en poids d'oligomères.

2.  Un procédé selon la revendication 1, dans lequel la charge contient un mélange d'oléfines légères.

3.  Un procédé selon la revendication 1 ou 2, dans lequel les oléfines contiennent de 2 à 7 atomes de carbone.

4.  Un procédé selon l'une quelconque des revendications précédentes dans lequel la charge contient de l'éthylène, du propylène, un butène, un pentène, un hexane et/ou un heptène.

5.  Un procédé selon l'une quelconque des revendications précédentes dans lequel la charge est un effluent gazeux d'une unité de fabrication de gaz contenant de l'éthylène et du propylène.

6.  Un procédé selon l'une quelconque des revendications précédentes dans lequel la charge est un effluent gazeux d'une unité de craquage de naphta contenant des oléfines légères.

7.  Un procédé selon l'une quelconque des revendications 1 à 4, dans lequel la charge est une essence légère craquée par voie catalytique contenant des pentènes, des hexènes et des heptènes.

8.  Un procédé selon l'une quelconque des revendications précédentes dans lequel la température est comprise entre 120 °C et 220 °C.

9.  Un procédé selon la revendication 8, dans lequel la température est comprise entre 140 °C et 200 °C.

10. Un procédé selon l'une quelconque des revendications précédentes dans lequel la pression est comprise entre 15 et 250 atmosphères (bar).

11. Un procédé selon la revendication 10, dans lequel la pression est comprise entre 20 et 200 atmosphères (bar).

12. Un procédé selon l'une quelconque des revendications précédentes dans lequel le rapport molaire eau/oléfine totale est compris entre 0,1 et 0,9.

13. Un procédé selon la revendication 12, dans lequel le rapport molaire eau/oléfine totale est comprise entre 0,2 et 0,8.

14. Un procédé selon l'une quelconque des revendications précédentes dans lequel le rapport de la structure silice/alumine de la zéolite bêta est supérieur à 20.

15. Un procédé selon l'une quelconque des revendications précédentes dans lequel le rapport de la structure silice/alumine de la zéolite bêta est supérieur à 200.

16. Un procédé selon l'une quelconque des revendications précédentes dans lequel la zéolite bêta est associée à un liant.

17. Un procédé selon la revendication 16, dans lequel le liant est la silice et/ou l'alumine.

18. Un procédé selon l'une quelconque des revendications précédentes dans lequel la zéolite bêta est associée avec au moins une quantité facilitant l'extrusion d'un liant à base d'un oxyde réfractaire de faible acidité, à l'état colloïdal, qui est sensiblement exempt de sel basique ou de base d'un métal alcalin.

**19.** Un procédé selon l'une quelconque des revendications précédentes dans lequel le catalyseur contient en outre un co-catalyseur efficace pour l'hydratation de l'oléfine légère.

**20.** Un procédé selon l'une quelconque des revendications précédentes dans lequel l'oléfine n'ayant pas réagi est recyclée.

**21.** Un procédé selon l'une quelconque des revendications précédentes dans lequel le produit ne contient pas plus de 10% en poids d'oligomères.

**22.** Un procédé selon l'une quelconque des revendications précédentes dans lequel la charge contient au moins 20% en poids de propylène.

**23.** Un procédé selon la revendication 22, dans lequel la charge est un courant d'oléfine légère provenant d'une unité FCC d'une raffinerie.